# EUROPEAN PATENT APPLICATION

(11) **EP 2 939 623 A1**
(43) Date of publication of application: **04.11.2015**
(21) Application number: 15172864.9
(22) Date of filing: 06.02.2012
(51) Int. Cl.: A61B 17/70

(54) **HIGH ANGULATION POLYAXIAL BONE ANCHORING DEVICE**

(30) Priority: 27.10.2011 US 201161552019 P
(62) Divisional of application: 12154115.5
(71) Applicant: Biedermann Technologies GmbH & Co. KG, 78166 Donaueschingen (DE)
(72) Inventor: Biedermann, Markku, Miami, FL 33131 (US); Santiago, José, Miami, FL 33131 (US)
(74) Representative: Prüfer & Partner GbR European Patent Attorneys

(57) **Abstract**

A polyaxial bone anchoring device is provided including
a bone anchoring element (1) having a shank (2) for anchoring in a bone or a vertebra and a head (3);
a receiving part (4, 4') for receiving a rod (5) to be connected to the bone anchoring element (1), the receiving part having a first end (4a) and an opposite second end (4b), a central axis (C) passing through the two ends, a channel for receiving the rod (5), a coaxial first bore (9) at the first end (4a) and a coaxial second bore (12) at the second end, that is in communication with the first bore (9);
an insert piece (6, 6', 6") that can be assembled and mounted to the receiving part (4, 4'),
the insert piece insert piece (6, 6', 6") having a seat (18) for the head of the bone anchoring element (1) to permit pivoting of the head relative to the seat,
wherein the insert piece (6, 6', 6") projects out of the second bore (12) and has a bound-ing edge at an end (6b) opposite to the first bore (9) with an opening (17) that is smaller than that of the head to prevent passage of the head therethrough; and
wherein the bounding edge is configured to permit the bone anchoring element (1) to pivot at a larger angle at a first location of the bounding edge than at another location of the bounding edge relative to the central axis (C); and
wherein the insert piece (6, 6', 6") is rotatable with respect to the receiving part and wherein the receiving part (4, 4') has a first flat surface portion (13) that is substantially perpendicular to the central axis (C) for supporting the insert piece (6, 6', 6") and the insert piece (6, 6', 6") has a corresponding second flat surface portion (15a) facing the first flat surface portion (13).

## Description

The invention relates to a polyaxial bone anchoring device that allows pivoting of the bone anchoring element at high angles. The device includes a bone anchoring element having a shank for anchoring in a bone or a vertebra and a head and a receiving part for receiving a rod to be connected to the bone anchoring element. An insert piece is rotatably mounted to the receiving part. The insert piece has a seat for the head of the bone anchoring element and is configured to permit the bone anchoring element to pivot at a larger angle at a first location than at another location. The insert piece is supported in the receiving part by a flat surface portion that engages a corresponding flat surface portion of the insert piece.

A polyaxial bone anchoring device with an enlarged pivot angle is described in US 6,736,820. This bone anchoring device comprises a bone screw and a receiving part with a seat for the head of the bone screw. The screw member can be pivoted to at least one side by an enlarged angle, because the edge bounding the free end of the receiving part is of asymmetric construction. In a modified embodiment, an insert piece is provided, which has a spherical bottom as a seat for the head of the screw member.

US 2005/0154391 A1 describes a bone anchor assembly including a bone anchor having a distal shaft configured to engage bone and a proximal member. The proximal member may have a first section and a second section coupled to at least a portion of the bone anchor. The second section may be movably connected to the first section to facilitate relative rotation of the first section and the second section.

US 2007/0118123 A1 describes a polyaxial bone anchor with increased angulation. The polyaxial bone anchor has a locking element shaped and configured to allow an anchoring member, e. g. a screw or a hook, to polyaxially rotate at large angles about a central axis of the bone anchor before compression locking the anchoring member within an anchor head.

It is the object of the invention to provide a polyaxial bone anchoring device with enlarged pivot angle that allows even greater pivot angles while simultaneously providing safe fixation. The object is solved by a polyaxial bone anchoring device according to claim 1. Further developments are given in the dependent claims.

The polyaxial bone anchoring device provides for a safe fixation, because the cooperating surfaces of the support of the insert piece are flat. Therefore, high pressure acting onto the head can be applied. Because the parts are mainly lathe parts, the manufacturing is simple and cost-effective. The bone anchoring device is also easy to assemble.

The bone anchoring device provides for high angulation with pivot angles between greater than 30° up to 100° (corresponding to a total range of motion of greater than 60° up to 200°). Due to the high angles, the bone anchoring device can also be used for sacral fixation.

The bone anchoring device can be provided as modular system with different insert pieces that can be used interchangeably to provide a wide range of high angulation. Due to the modularity, less inventory is needed that reduces the costs further.

Further features and advantages will become apparent in the description of embodiments by means of the accompanying drawings. In the drawings:
- Fig. 1:: shows a perspective exploded view of the polyaxial bone anchoring device according to a first embodiment.
- Fig. 2:: shows a perspective view of the polyaxial bone anchoring device of Fig. 1 in an assembled state.
- Fig. 3:: shows a cross-sectional view of the polyaxial bone anchoring device according to the first embodiment, wherein the section has been taken in a plane containing the rod axis and wherein the bone anchoring element is pivoted at a first pivot angle to one side.
- Fig. 4:: shows a cross-sectional view of the polyaxial bone anchoring device according to the first embodiment, wherein the section has been taken in a plane containing the rod axis and wherein the bone anchoring element is pivoted to a second pivot angle at the opposite side compared to Fig. 3.
- Fig. 5:: shows a perspective view of the receiving part of the polyaxial bone anchoring device according to the first embodiment.
- Fig. 6:: shows a side view of the receiving part of Fig. 5.
- Fig. 7:: shows a cross-sectional view of the receiving part of Fig. 5, wherein the section has been taken in a plane containing the rod axis.
- Fig. 8:: shows a top view of the receiving part of Fig. 5.
- Fig. 9:: shows a perspective view of the insert piece of the polyaxial bone anchoring device according to the first embodiment.
- Fig. 10:: shows a cross-sectional view of the insert piece of Fig. 9, wherein the section has been taken along line A-A in Fig. 9.
- Fig. 11:: shows a top view of the insert piece of Figs. 9 and 10.
- Fig. 12:: shows a perspective view of the pressure element of the polyaxial bone anchoring device according to the first embodiment.
- Fig. 13:: shows another perspective view from below of the pressure element of Fig. 12.
- Fig. 14:: shows a cross-sectional view of the pressure element of the polyaxial bone anchoring device according to the first embodiment, wherein the section has been taken in a plane containing the rod axis.
- Fig. 15:: shows a side view of the pressure element of Fig. 12.
- Fig. 16:: shows a top view of the pressure element of Fig. 12.
- Fig. 17:: shows a perspective view of the polyaxial bone anchoring device according to a second embodiment in an assembled state.
- Fig. 18:: shows another perspective view from below of the polyaxial bone anchoring device according to the second embodiment.
- Fig. 19:: shows a cross-sectional view of the polyaxial bone anchoring device according to the second embodiment.

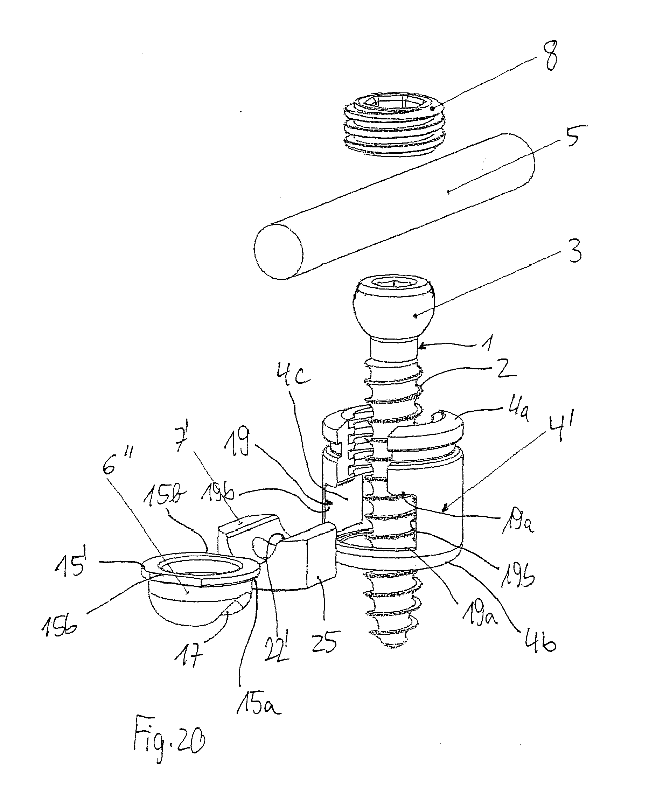

- Fig. 20:: shows an exploded perspective of a third embodiment of the polyaxial bone anchoring device.
- Figs. 21 a) to 21d): show steps of assembling the polyaxial bone anchoring device according to the third embodiment.

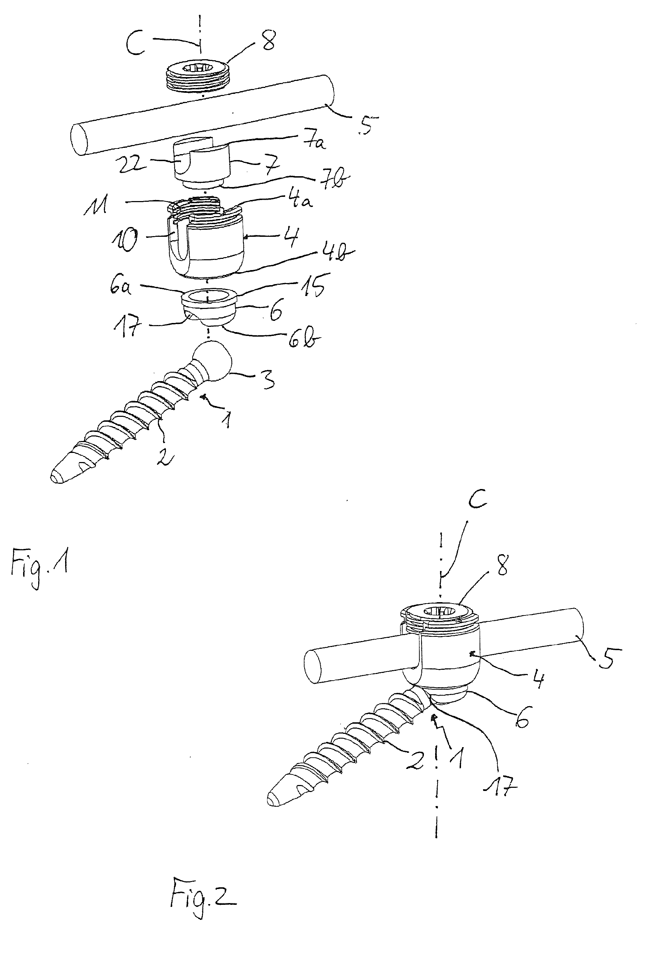

Referring to Figs. 1 to 4, a polyaxial bone anchoring device according to a first embodiment includes a bone anchoring element 1 in the form of a bone screw having a shank 2 with a threaded section and a head 3. The head 3 has a spherically-shaped outer surface portion and comprises a recess 3 a at its free end for engagement with a driver. The bone anchoring device further includes a receiving part 4 for receiving a rod 5 to be coupled to the bone anchoring element 1. In the receiving part 4, an insert piece 6 providing a seat for the head 3 and a pressure member 7 for exerting pressure onto the head 3 of the bone anchoring element 1 are arranged. Furthermore, a fixation element in the form of a fixation screw or set screw 8 is provided for securing and fixing the rod 5 in the receiving part 4 and also for locking the bone screw in place in the receiving part..

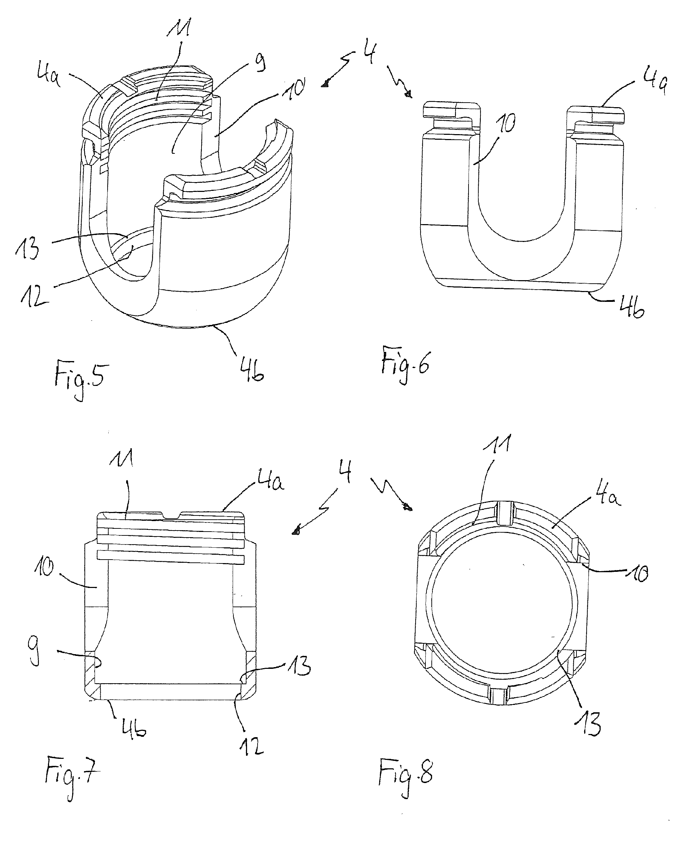

As can be seen best in Figs. 5 to 8, the receiving part 4 has a top end 4a and a bottom end 4b, a central axis defining the central axis C of the polyaxial bone anchoring device and a coaxial first bore 9 extending from the top end 4a in the direction of the bottom end 4b. Adjacent to the top end 4a, a substantially U-shaped recess 10 is provided that forms a channel for receiving the rod 5. By means of the recess 10, two free legs are formed, that are provided with an internal thread 11 cooperating with the fixation screw 8.

At the second end 4b, a coaxial second bore 12 is provided, that is in communication with the first bore 9 and the diameter of which is smaller than that of the first bore 9. At the transition of the first bore 9 to the second bore 12, a support surface 13 is formed that extends perpendicular to the central axis C, so that the support surface 13 is substantially flat. In the embodiment shown, the support surface 13 is an annular shoulder.

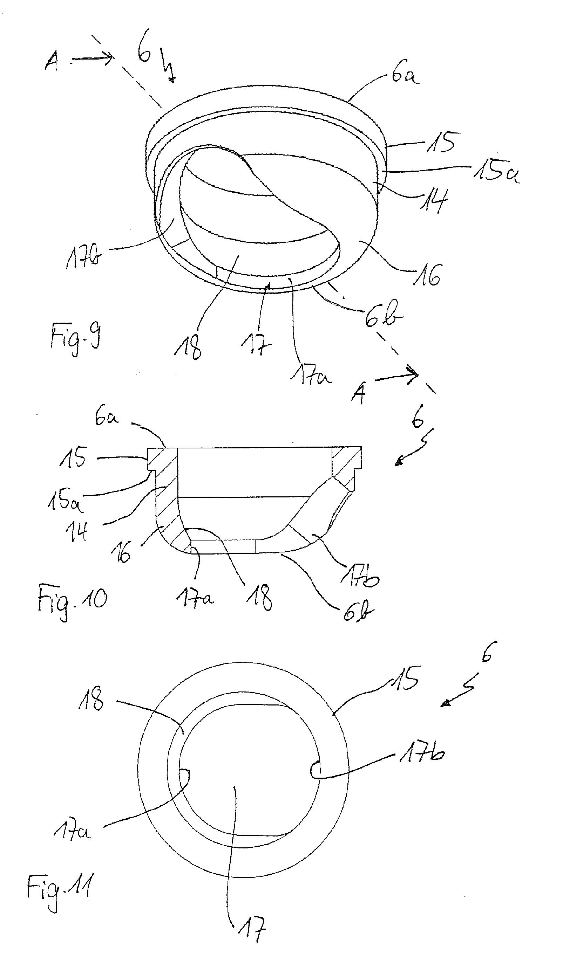

As can be seen in Figs. 9 to 11, the insert piece 6 is a substantially cylindrical part with a first end 6a and an opposite second 6b, a hollow cylindrical portion 14 with an inner diameter that is larger than the diameter of the head 3, so that the head 3 of the screw of the anchoring element 1 can be guided therethrough. The outer diameter of the hollow cylindrical portion 14 is slightly smaller than the inner diameter of the coaxial first bore 12 of the receiving part 4. Adjacent the first end 6a, an annular projection 15 is provided that has a flat lower side 15a extending perpendicular to the central axis C and that cooperates with the support surface 13 in the receiving part 4. The outer diameter of the annular projection 15 is slightly smaller than the outer diameter of the coaxial first bore 9 and larger than the diameter of the coaxial second bore 12 of the receiving part 4.

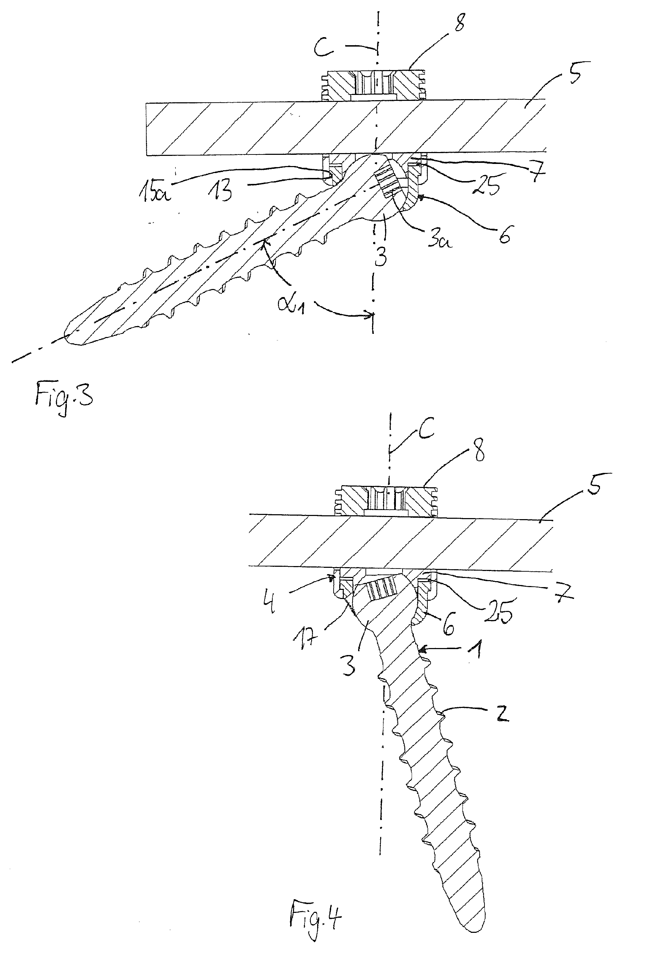

The insert piece 6 further has a lower portion 16 with an elongate opening 17 at the second end 6b and with a hollow spherically-shaped section 18 providing a seat for the head 3 of the anchoring element. The outer surface of the lower portion 16 has the shape of a spherical segment. However, it is not necessary limited to such a shape. The hollow spherically-shaped section 18 is in communication with the hollow cylindrical portion 14. The opening 17 has a partially spherical first portion 17a as shown in Fig. 10 and an elongate second portion 17b extending from the lower portion 16 into the hollow cylindrical portion 14. The diameter of the opening is larger than the diameter of the threaded shank 2 and smaller than the diameter of the head 3, so that the threaded shank 2 of the bone anchoring element 1 can extend through the opening 17, but the head 3 can not pass through the opening 17. By means of the opening 17, an axis P for a maximum pivot angle α₁ is defined by the shank axis of the threaded shank 2 when the bone anchoring element is inserted and pivoted until the shank 2 abuts against the end of the elongate second portion 17b of the opening 17 as shown in Fig. 3. The edge bounding the opening 17 is an edge that allows the bone anchoring element to pivot at a larger angle when its shaft extends into the elongate portion 17b of the opening 17 (Fig. 3) compared to a pivot angle when the shank is at another location, for example opposite to the elongate portion 17b (Fig. 4).

The axial length of the hollow cylindrical portion 14 and of the lower portion 16 is such that when the insert piece is inserted into the receiving part 4 as shown in Figs. 3 and 4, the end of the elongate portion 17b of the opening 17 is flush with or projects slightly out of the second end 4b of the receiving part 4.

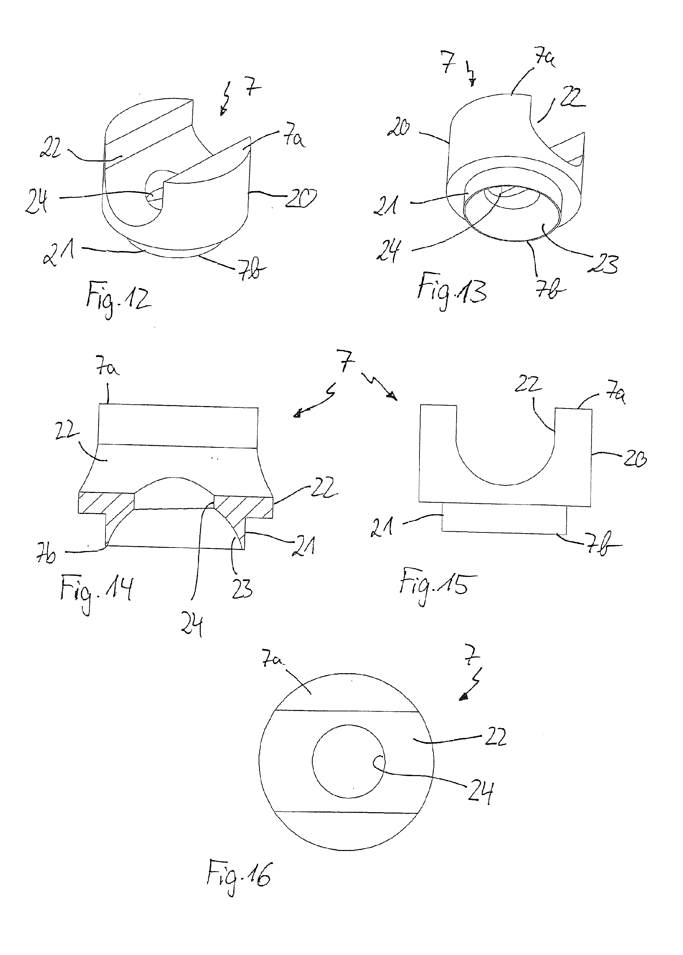

The pressure member will be explained with reference to Figs. 12 to 16. The pressure member 7 has a first end 7a and an opposite second end 7b and comprises a first cylindrical portion 20 including the first end 7a and a second cylindrical portion 21 including the second end 7b. The outer diameter of the first cylindrical portion 20 is slightly smaller than the inner diameter of the coaxial first bore 9 and the outer diameter of the second cylindrical portion is smaller than the outer diameter of the first cylindrical portion 20. Adjacent the first end 7a, a substantially U-shaped recess 22 is provided that is configured to accommodate the rod 5 therein. Adjacent the second end 7b, a spherical recess 23 is provided with a radius of the sphere matching the radius of the head 3 of the bone anchoring element 1. Furthermore, a coaxial bore 24 extends between the first end 7a and the second end 7b to allow access to the head 3 with a driver.

As can be seen in Figs. 3 and 4, the dimensions of the first cylindrical portion 20 and the second cylindrical portion 21 and the U-shaped recess 22 are such that when the insert piece 6 together with the bone anchoring element 1 and the pressure member 7 and the rod 5 are inserted into the receiving part 4, the pressure member 7 contacts only the head 3 while there is a gap 25 between the first cylindrical portion 20 of the pressure member 7 and the first end 6a of the insert piece 6. Therefore, pressure exerted onto the pressure member is fully transmitted onto the head 3 and presses the head 3 into the seat 18. The rod 5 projects out of the U-shaped recess so that the fixation element 8 engages the rod 5.

The parts of the bone anchoring device are made of a biocompatible material, such as titanium, stainless steel, a biocompatible alloy, for example a nickel titanium Ni-Ti alloy, especially Nitinol, or of a biocompatible plastic material, such as, for example, polyetheretherketone (PEEK). The parts can be made all of the same materials or of different materials.

The bone anchoring device can be assembled as follows. First, the insert piece 6 is introduced into the receiving part 4. Then the bone anchoring element 1 is inserted into the receiving part 4 such that the shank 2 extends through the insert piece. When the bone anchoring element 1 is fully inserted, the head 3 rests in the seat 18 in the insert piece 6. Thereafter, the pressure member 7 can be inserted and may be preliminarily fixed through crimping via crimp bores in the receiving part 4 (not shown). The preliminary fixation of the pressure member 7 may be such that the pressure member exerts a slight pressure onto the head 3 which holds the receiving part 4 in a preliminary position.

Due to the symmetric design of the upper portion 14, 15 of the insert piece, the insert piece can be inserted in any orientation of the opening 17 into the receiving part.

In use, the bone anchoring element is screwed into the bone or a vertebra, while the central axis of the bone anchoring element 1 and of the receiving part 4 are substantially aligned. Then, the receiving part 4 is pivoted with respect to the bone anchoring element 1. If required, the receiving part can be pivoted with respect to the bone anchoring element to the maximum pivot angle α₁ until the shank 2 abuts against the end of the elongate portion 17b of the opening 17 of the insert piece. The orientation of the U-shaped recess 12 of the receiving part can be aligned by rotating the receiving part with respect to the insert piece 6. Because the cooperating surfaces of the insert piece and the receiving part are flat surfaces, the friction between these surfaces is enhanced. Therefore, a preliminary orientation of the insert piece with respect to the receiving part can be maintained by friction. In addition, the corresponding surfaces can have a mechanical structure, such as a roughened structure, to enhance the friction. The insert piece is then rotated by application of a force greater than the friction force. This allows to precisely adjust the orientation of the receiving part.

A plurality of bone anchoring elements are anchored in the bone and their receiving parts are aligned and finally the rod 5 is inserted. Inserting and tightening the fixation element 8 locks the head 3 and the rod 5.

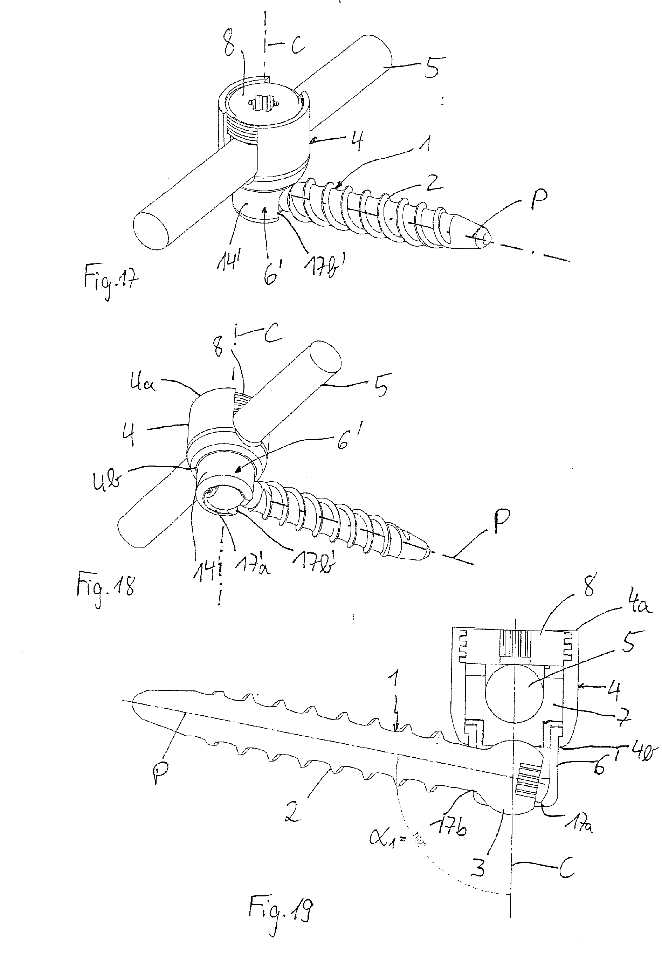

Referring to Figs. 17 to 19, a second embodiment of the bone anchoring device is shown. The second embodiment differs from the first embodiment with respect to the insert piece and the pressure member. All other parts are the same and characterized with the same reference numerals and the description thereof is not repeated. The insert piece 6' has a first end 6a' and a second end 6b' and a cylindrical portion 14' that is larger in axial direction. Therefore, the insert piece 6' projects farther outward of the receiving part compared to the insert piece 6 according to the first embodiment. The opening 17' has a circular portion 17a' at the second end 6b' of the insert piece 6' and a substantially U-shaped portion 17b' extending into the wall of the cylindrical portion 14'. Because the cylindrical portion 14' and the opening portion 17b' are longer in axial direction, it is possible to pivot the bone anchoring element relative to the receiving part to angles of 90° or more, for example to 100° as shown in the Figs.

With the polyaxial bone anchoring device described above, a modular system can be provided that includes a receiving part 4 and a bone anchoring element 1 and at least two insert pieces 6, 6' that have a different length of the cylindrical portion 14, 14' and of the opening 17b, 17b' to allow pivoting of the bone anchoring element to two different maximum pivot angles.

A polyaxial bone anchoring device according to the third embodiment differs from the previous embodiments in that the receiving part 4' has a substantially rectangular opening 19 in its wall. The opening 19 has long sides 19a extending in the circumferential direction and short sides 19b extending in axial direction. At its inner wall, the receiving part 4' has two opposite flat portions 4c configured to cooperate with the pressure member 7' described below. The flat portions are located on either side of the opening 19.

The insert piece 6" has cylindrical projection 15", where on opposite side two portions are cut away so that two opposite flat sections 15b remain. The diameter of the projection 15 at the rounded sides is slightly smaller than the inner diameter of the opening 19 at the long sides 19a. Similarly, the pressure member 7' has two flat portions 25 on opposite sides of the outer surface at either end of a cylindrical recess 22'. The diameter of the pressure member 7' from one flat side 25 to the opposite flat side 25 is smaller than the length of the long side 19a of the opening. By means of this, it is possible to insert the insert piece 6" and the pressure member 7' through the opening 19 into the receiving part.

By the flattened portions on the pressure member 7' and the insert piece 6", the overall dimensions of the bone anchoring device, in particular with respect to the receiving part, can be reduced.

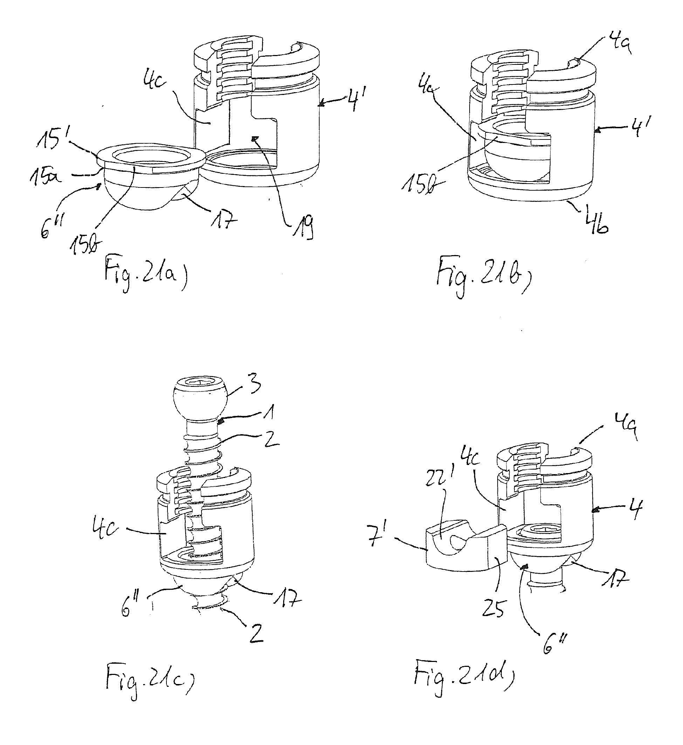

The assembly of the bone anchoring device is shown in Figs. 21a)-21d). First, the insert piece 6" is inserted through the opening 19 and moved downward until the flat surface 15a at the lower side of projection 15 is supported on the flat surface 13a in the receiving part. Then, as shown in Fig. 21 c), the bone anchoring element 1 is introduced from the first end 4a of the receiving part 4' until its head 3 is seated in the seat 18 of the insert piece 6". Thereafter, the pressure member 7' is inserted through the opening. The modular system allows to provide various insert pieces with various angles for the maximum pivot angle and to combine a suitable insertion piece with the receiving part.

Because the pressure member 7' has two opposite flat portions 25 that cooperate with the flat portions 4c at the inner wall of the receiving part, the pressure member 7' is prevented from rotation.

Further modifications of the embodiments described are conceivable. For example, for the bone anchoring element, all kinds of anchoring elements can be used, for example, screws, canulated screws, or nails. The head and the shank may also be separate parts that are connectable to each other.

Other kinds of locking devices including outer nuts, outer caps, bayonet locking devices, are possible. The locking device can also be a two-part locking device having one locking element that locks the head and another locking element that locks the rod.

While the bounding edge of the insert piece is asymmetric by means of an elongate opening, an asymmetry that allows a larger pivot angle to one side can also be achieved by an inclined lower edge of the insert piece.

## Claims

1. A polyaxial bone anchoring device including
a bone anchoring element (1) having a shank (2) for anchoring in a bone or a vertebra and a head (3);
a receiving part (4, 4') for receiving a rod (5) to be connected to the bone anchoring element (1), the receiving part having a first end (4a) and an opposite second end (4b), a central axis (C) passing through the two ends, a channel for receiving the rod (5), a coaxial first bore (9) at the first end (4a) and a coaxial second bore (12) at the second end, that is in communication with the first bore (9);
an insert piece (6, 6', 6") that can be assembled and mounted into the receiving part (4, 4'),
the insert piece (6, 6', 6") having a seat (18) for the head of the bone anchoring element (1) to permit pivoting of the head relative to the seat,
wherein the insert piece (6, 6', 6") projects out of the second bore (12) and has a bounding edge at an end (6b) opposite to the first bore (9) with an opening (17) that is smaller than that of the head to prevent passage of the head therethrough; and
wherein the bounding edge is configured to permit the bone anchoring element (1) to pivot at a larger angle at a first location of the bounding edge than at another location of the bounding edge relative to the central axis (C); and
wherein the insert piece (6, 6', 6") is rotatable with respect to the receiving part and wherein the receiving part (4, 4') has a first flat surface portion (13) that is substantially perpendicular to the central axis (C) for supporting the insert piece (6, 6', 6") and the insert piece (6, 6', 6") has a corresponding second flat surface portion (15a) facing the first flat surface portion (13).

2. The polyaxial bone anchoring device of claim 1, wherein the first flat surface portion (13) extends circumferentially around the central axis (C).

3. The polyaxial bone anchoring device of claim 1 or 2, wherein the second flat surface portion (15a) extends circumferentially around the central axis (C).

4. The polyaxial bone anchoring device of one of claims 1 to 3, wherein the first flat surface portion (13) is comprised in an annular projection projecting from an inner wall of the receiving part into the first bore (9).

5. The polyaxial bone anchoring device of one of claims 1 to 4, wherein the second flat surface portion (15a) is comprised in an annular projection projecting outward from the insert piece.

6. The polyaxial bone anchoring device of one of claims 1 to 5, wherein the insert piece (6, 6', 6") has a first end portion (15, 14) that is rotationally symmetric and a second end portion (16) that defines an asymmetric bounding edge.

7. The polyaxial bone anchoring device of one of claims 1 to 6, wherein the insert piece has a recess (17) through which the shank of the bone anchoring element extends and that is asymmetric with respect to the central axis (C).

8. The polyaxial bone anchoring device of one of claims 1 to 7, wherein the cooperating flat surface portions (13, 15a) of the receiving part and the insert piece (6, 6', 6") are configured to generate a friction force between them that holds the insert piece in a specific rotational position with respect to the receiving part, wherein the position can be changed by applying a force greater than the friction force.

9. The polyaxial bone anchoring device of one of claims 1 to 7, further comprising a pressure element (7, 7') that is configured to move in the receiving part in a direction of the axis of the first bore and that exerts pressure onto the head.

10. The polyaxial bone anchoring device of one of claims 1 to 9, further comprising a fixation element (8) for fixation of the rod.

11. The polyaxial bone anchoring device of one of claims 1 to 10, wherein the insert piece (6, 6', 6") comprises a hollow cylindrical portion (14, 14') with an inner diameter that is larger than the diameter of the head (3).

12. The polyaxial bone anchoring device of claim 11, wherein the insert piece (6, 6', 6") has a lower portion (16) with an elongate opening (17, 17') and with a hollow spherically-shaped section (18) providing a seat for the head (3).

13. The polyaxial bone anchoring device of one of claims 4 to 12, wherein the receiving part comprises a coaxial first bore extending from the first end (4a) in the direction of the second end (4b) and wherein the annular projection (15) has an outer diameter that is slightly smaller than the inner diameter of the first bore (9).

14. The polyaxial bone anchoring device of one of claims 1 to 13, wherein a preliminary orientation of the insert piece (6, 6', 6") and the receiving part (4, 4', 4") is maintained by friction between the cooperating surface portions (13, 15a) of the insert piece (6, 6', 6") and the receiving part (4, 4').

15. A modular polyaxial bone anchoring system including
a bone anchoring element (1) having a shank (2) for anchoring in a bone or a vertebra and a head (3);
a receiving part (4, 4') for receiving a rod (5) to be connected to the bone anchoring element (1), the receiving part having a first end (4a) and an opposite second end (4b), a central axis ( C) passing through the two ends, a channel for receiving the rod (5), a coaxial first bore (9) at the first end (4a) and a coaxial second bore (12) at the second end, that is in communication with the first bore (12);
and a first insert piece (6) that can be assembled and mounted to the receiving part (4, 4'),
the first insert piece insert piece (6) projecting out of the second bore (12) and having a bounding edge at an end opposite to the first bore with an opening (17) that is smaller than that of the head to prevent passage of the head therethrough; and
wherein the bounding edge is configured to permit the bone screw to pivot at a first larger angle at a first location of the bounding edge than at another location of the bounding edge, relative to the axis of the first bore; and
a second insert piece (6') that can be assembled and mounted to the receiving part (4, 4'),
the second insert piece insert piece (6') projecting out of the second bore (12) and having a bounding edge at an end opposite to the first bore with an opening (17') that is smaller than that of the head to prevent passage of the head therethrough; and
wherein the bounding edge is configured to permit the bone screw to pivot at a second larger angle at a first location of the bounding edge than at another location of the bounding edge, relative to the axis of the first bore;
wherein the first insert piece (6) and the second insert piece (6') can be mounted interchangeably; and wherein preferably
the second insert piece (6') projects farther out of the second bore (12) than the first insert piece (6).
